# EUROPEAN PATENT APPLICATION

(11) **EP 4 759 286 A1**
(43) Date of publication of application: **17.06.2026**
(21) Application number: 24219979.2
(22) Date of filing: 13.12.2024
(51) Int. Cl.: A61G 13/00, A61G 13/06, A61G 13/08, A61G 13/12

(54) **ADJUSTABLE OPERATING TABLE**

(71) Applicant: Baxter Medical Systems GmbH + Co. KG, 07318 Saalfeld (DE)
(72) Inventor: GRIESEL, Andre, 07318 Saalfeld (DE); TOEPEL, Norman, 07318 Saalfeld (DE)
(74) Representative: Reddie & Grose LLP

(57) **Abstract**

The present disclosure relates to an operating table for adjusting the position of a patient, for example such that their kidney or thorax region are elevated. The present disclosure further relates to a method of adjusting an operating table. The operating table comprises an operating table surface for supporting a patient; and a column, the operating table surface being connected to the column. The operating table surface comprises a seat section, comprising: a first seat portion having a first longitudinal end and a second longitudinal end; and a second seat portion having a first longitudinal end and a second longitudinal end. The second longitudinal end of the first seat portion is pivotably coupled to the first longitudinal end of the second seat portion. The method comprises configuring the operating table surface in a first pre-defined operating table surface configuration in which the operating table surface is substantially flat; and, configuring the operating table surface in a second pre-defined operating table surface configuration in which the first seat portion and the second seat portion are configured such that a patient being supported on the operating table has their kidney region elevated relative to their pelvic region.

## Description

### Technical Field

The present disclosure relates to an operating table for adjusting the position of a patient, for example such that their kidney or thorax region are elevated. The present disclosure further relates to a method of adjusting an operating table.

### Background

As minimally invasive and robot-assisted surgeries have become increasingly common, the demands on operating table functionality have also grown. Typical surgical procedures require either lateral patient positioning with the patient's kidney region elevated or supine patient positioning with the patient's thorax region elevated. This allows improved access to the point of interest within the patient.

In order to provide improved access, it is known to adjust the position of an operating table to achieve desired patient positioning during surgical procedures. Conventional operating tables typically have a foot section, a seat section, a back section, and a head section. For the typical surgical procedures outlined above, the foot and seat section are normally tilted in a downward fashion, that is to say in the reverse Trendelenburg direction, with the back section moved downwards.

Taking robotic surgery into account, the present inventors have appreciated that these downward tilting foot, seat and back sections can present challenges. In particular, as the table is tilted, any attached accessories, particularly those attached below the table, may come into close proximity with, or may even collide with, the table base or other surrounding equipment.

Therefore, the present inventors have appreciated that there is a desire for an improved operating table for use within minimally invasive and robotically-assisted surgeries which involve lateral positioning with a patient's kidney region elevated or supine positioning with a patient's thorax region elevated.

### Summary of the Disclosure

Examples described herein provide an operating table for adjusting the position of the patient as defined in the appended independent claims, to which reference should now be made. Preferred or advantageous features of the disclosure are set out in the dependent claims.

According to a first aspect of the present disclosure, there is provided an operating table for adjusting the position of the patient. The operating table comprises an operating surface for supporting a patient. The operating table further comprises a column. The operating table surface is connected to the column. The operating table surface comprises a seat section. The seat section comprises a first seat portion having a first longitudinal end and second longitudinal end. The seat section further comprises a second seat portion having a first longitudinal end and second longitudinal end. The second longitudinal end of the first seat portion is pivotally coupled to the first end of the second seat portion.

The inventors have identified that by splitting the standard seat section of an operating table into two parts, a first seat portion and a second seat portion, a user might more easily adjust the position of a patient such that their kidney or thorax region are elevated. The user may be, but not limited to, a hospital technician, a user, or a doctor. Additionally, the patient can be advantageously positioned without the first or second seat portion interfering with any elements below the operating table, such as robotic surgery equipment.

The first seat portion and second portion may be arranged such that their total length is no more than about 125%, no more than about 110%, or no more than about 105% of the standard length for an operating table seat section. In one particular example, the total length of the first seat portion and the second seat portion is about 100% of the standard length. As used herein, the "standard length" of an operating table is that of previous corresponding models of operating table. In this way, the advantages of the present operating table can be provided without the requirement for significant modification of the "standard" operating table features, or use practices.

The inventors have advantageously split the standard seat section of an operating table into two portions whilst substantially maintaining the length of a standard seat section of an operating table. This provision enables an operating table with the advantages associated with the split seat section to be provided whilst maintaining the usability of a standard operating table.

The operating table may further comprise a back section having a first and second longitudinal end. The first longitudinal end of the back section is to be pivotally coupled to the to the second longitudinal end of the second seat portion. The operating table may comprise a second seat portion actuator configured to pivot the second seat portion about the second longitudinal end of the first seat portion. The operating table may further comprise a back section actuator configured to pivot the back section about the second longitudinal end of the second seat portion.

The provision of the second seat portion actuator and the back section actuator may advantageously enable the user to more easily position the portions of the operating table surface.

The operating table surface may comprise a head section. The head section is to be coupled to the second longitudinal end of the back section. The head section may advantageously support the patient's head when a patient is on the operating table surface. The head section may be removably detachable from the back section.

The head section may be pivotably coupled to the second longitudinal end of the back section. The operating table may further comprise a head section actuator configured to pivot the head section about the second longitudinal end of the back section. The head section actuator may advantageously automate the angle at which the patient's head is supported.

The operating table surface may further comprise a foot section. The foot section is to be coupled to the first longitudinal end of the first seat portion. The foot section may be removably detachable from the first seat portion. The foot section may advantageously support the patient's lower leg sections when a patient is on the operating table surface.

The foot section may be pivotably coupled to the first longitudinal end of the first seat portion. The operating table may further comprise a foot section actuator configured to pivot the foot section about the first longitudinal end of the first seat portion. A foot section actuator may advantageously automate the angle at which the patient's legs are supported.

Optionally, the pivotable coupling between any portion of the operating table according to the present disclosure may be formed as a joint. The joint may be a pin joint or a hinge joint.

The connection between the first seat portion and the column may be configured such that the longitudinal axis of the first seat portion is perpendicular to the longitudinal axis of the column.

The operating table may further comprise a controller. The controller is configured to control one or more of the actuators to position the operating table surface. The implementation of a controller into the operating table may advantageously enable greater control of the positioning of the operating table surface.

The one or more actuators may comprise at least one sensor to measure the rotational position of the one or more actuators. The at least one sensor may be an encoder sensor, a resolver sensor, a rotary potentiometer sensor, or a hall effect sensor. The controller may be configured to receive positional feedback from the at least one sensor. The implementation of the at least one sensor may advantageously improve the accuracy of the controller when controlling the one or more actuators.

The operating table may further comprise a virtual anti-collision plane. As used herein with reference to the present disclosure, the term "virtual anti-collision plane" refers to a plane that is orthogonal to the longitudinal axis of the column and intersecting the connection between the column and the operating table surface.

Alternatively, or in addition, the operating table may further comprise a head section virtual anti-collision plane and/or a foot section anti-collision plane. As used herein with reference to the present disclosure, the term "head section virtual anti-collision plane" refers to a plane that is orthogonal to the longitudinal axis of the column, extending longitudinally from the first longitudinal end of the first seat portion towards the longitudinal end of the head section, and intersecting the connection between the column and the operating table surface. As used herein with reference to the present disclosure, the term "foot section virtual anti-collision plane" refers to a plane that is orthogonal to the longitudinal axis of the column, extending longitudinally from the first longitudinal end of the back section towards the longitudinal end of the foot section, and intersecting the connection between the column and the operating table surface.

The virtual anti-collision plane may be selected according to the use case of the operating table.

The controller may be configured such that no portion of the operating table surface may cross at least the virtual anti-collision plane, the head section virtual anti-collision plane, or the foot section virtual anti-collision plane. The inventors have identified that by ensuring no portion of the operating table enters a virtual anti-collision plane, the present disclosure may advantageously enable robotic surgery equipment to move freely around the entire operating table.

The controller may comprise instructions for at least two pre-defined operating table surface configurations, a first pre-defined operating table surface configuration and second pre-defined operating table surface configuration. The controller is configured to cause the actuators to configure the operating table surface to be in the first pre-defined operating table surface configuration or the second pre-defined operating table surface configuration. As will be appreciated, the controller is preferably configured to enable the operating table surface to move in a continuous motion between the first pre-defined operating table surface configuration and the second pre-defined operating table surface configuration. The continuous motion may cause the back section to move in an opposite rotation, relative to the second seat portion, as compared to the rotation of the second seat portion, relative to the first seat portion. The rotational speed of the back section may be greater than the rotational speed of the second seat portion, optionally at least 1.5 times the rotational speed, and further optionally less than at least 2 times the rotational speed.

The "first pre-defined operating table surface configuration" may be a configuration in which the operating table surface is substantially flat.

The "second pre-defined operating table surface configuration" may be a configuration in which the first seat portion and the second seat portion are configured such that a patient being supported on the operating table has their kidney region or thorax region elevated relative to their pelvic region.

Normally, external features are added to a standard operating table such that the operating table may provide optimal patient positioning for different surgical procedures. However, the inventors have identified that the first and second pre-defined operating table surface configurations may advantageously enable the operating table to be used for more than just one surgical procedure without the need for adding external elements to the operating table.

The second pre-defined operating table surface configuration may be a configuration wherein the second seat portion is at an angle, relative to the first seat portion, of at least 110 degrees, at least 120 degrees, or at least 130 degrees.

The second pre-defined operating table surface configuration may be a configuration wherein the second seat portion is at an angle, relative to the first seat portion, of at most 170 degrees, at most 160 degrees, or at most 150 degrees.

For example, the second pre-defined operating table surface configuration may be a configuration wherein the second seat portion is at an angle, relative to the first seat portion, of between about 110 degrees and about 170 degrees, between about 120 degrees and 160 degrees, or between about 130 degrees and 150 degrees.

As used herein with reference to the present disclosure, any stated angle between two portions of the operating table according to the present disclosure are external angles measured between the top surface of one portion and the top surface of the other portion.

The second pre-defined operating table surface configuration may be a configuration wherein the back section is at an angle, relative to the first seat portion, of at most 230 degrees, at most 220 degrees, or at most 210 degrees. In one particular example, the angle is about 205 degrees.

The second pre-defined operating table surface configuration may be a configuration wherein the back section is at an angle, relative to the first seat portion, of at least 190 degrees, at least 195 degrees, or at least 200 degrees.

For example, the second pre-defined operating table surface configuration may be a configuration wherein the back section is at an angle, relative to the first seat portion, of between about 190 degrees and about 230 degrees, between about 195 degrees and 220 degrees, or between about 200 degrees and 210 degrees.

The inventors have identified that these angle ranges for the back section relative to the first seat portion are most suitable for elevating the kidney or thorax region of a patient on the operating table according to the present disclosure.

The controller may be configured to receive a first input and a second input. The controller may be further configured to receive at least one of: a third, fourth, fifth, sixth input, seventh input, and eighth input. The first input causes the controller to move the operating table towards the first pre-defined operating table surface configuration. The second input causes the controller to move the operating table towards the second pre-defined operating table surface configuration.

The first and second input may enable the user to adjust the position of the operating table surface to be in a configuration that is anywhere between, and including, the first pre-defined and second pre-defined operating table configuration. This provision advantageously allows the user to incrementally position the patient on the operating table surface such that they are in an optimal position.

The third input causes the controller to move the foot section towards a minimum angle, relative to the first seat portion, of at least 90 degrees, of at least 100 degrees, or at least 110 degrees. In the instance where the controller is configured such that no portion of the operating table crosses the head section virtual anti-collision plane, the fourth input causes the controller to move the foot section towards a maximum angle, relative to the first seat portion, of at most 255 degrees, at most 265 degrees, or at most 275 degrees. In the instance where the controller is configured such that no portion of the operating table crosses the foot section virtual anti-collision plane, the fourth input causes the controller to move the foot section towards a maximum angle, relative to the first seat portion, of at most 160 degrees, at most 170 degrees, or at most 180 degrees. Such an angle ensures that the foot section does not cross the foot section virtual anti-collision plane, and that the foot section is substantially parallel to the floor.

In the instance where the controller is configured such that no portion of the operating table crosses the head section virtual anti-collision plane, the fifth input causes the controller to move the head section towards a maximum angle, relative to the first seat potion, of at most 160 degrees, at most 170 degrees, or at most 180 degrees. Such an angle ensures that the head section does not cross the head section virtual anti-collision plane, and that the head section is substantially parallel to the floor. In the instance where the controller is configured such that no portion of the operating table crosses the foot section virtual anti-collision plane, the fifth input causes the controller to move the head section towards a maximum angle, relative to the first seat potion, of at most 255 degrees, at most 265 degrees, or at most 275 degrees. The sixth input causes the controller to move the head section increasingly towards a minimum angle of, relative to the first seat portion, of at least 150 degrees, at least 140 degrees, or at least 130 degrees.

The seventh input causes the controller to move the back section towards a minimum angle, relative to the first seat portion, of at least 160 degrees, 170 degrees, or at least 180 degrees. The eighth input causes the controller to move the back section towards a maximum angle, relative to the first seat portion, of at most 255 degrees, 245 degrees, or at most 235 degrees.

According to a second aspect of the present disclosure, there is provided a method of configuring an operating table for adjusting the position of a patient. The method may comprise configuring the operating table surface in a first pre-defined operating table surface configuration in which the operating table surface is substantially flat.

The method may further comprise configuring the operating table surface in a second pre-defined operating table surface configuration in which the first seat portion and the second seat portion are configured such that a patient being supported on the operating table has their kidney region or thorax region elevated relative to their pelvic region.

The method may advantageously enable precise and complex movements of an operating table and improved patient positioning during surgical procedures while reducing the risk of collisions with accessories and equipment.

The disclosure will be further described, by way of example only, with reference to the accompanying drawings in which:
Figure 1a shows a side view of an operating table 100 according to the present disclosure, wherein the operating table 100 is in an first pre-defined operating table surface configuration.
Figure 1b shows a side view of the operating table 100 according to the present disclosure, wherein the operating table 100 is in a second pre-defined operating table configuration.
Figure 2 shows a side view of the operating table 100 of Figure 1a and Figure 1b, wherein the main features of the operating table are depicted.
Figure 3 illustrates a block diagram of the control system and user interface for the operating table 100.
Figure 4a shows a side view of the operating table 100 of Figure 1 and a respective virtual anti-collision plane 400A.
Figure 4b shows a side view of the operating table 100 of Figure 1 and a respective head section virtual anti-collision plane 400B.
Figure 4c shows a side view of the operating table 100 of Figure 1 and a respective foot section virtual anti-collision plane 400C.

### DETAIL DESCRIPTION OF DRAWINGS

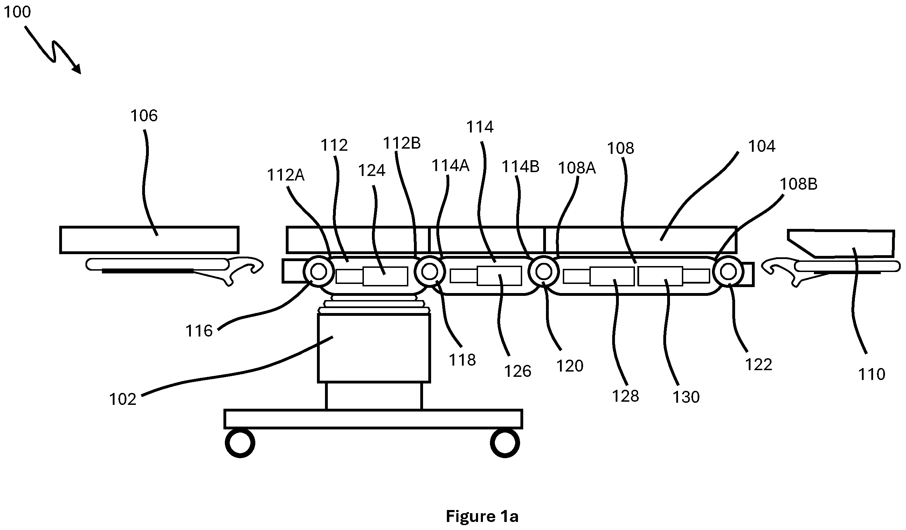

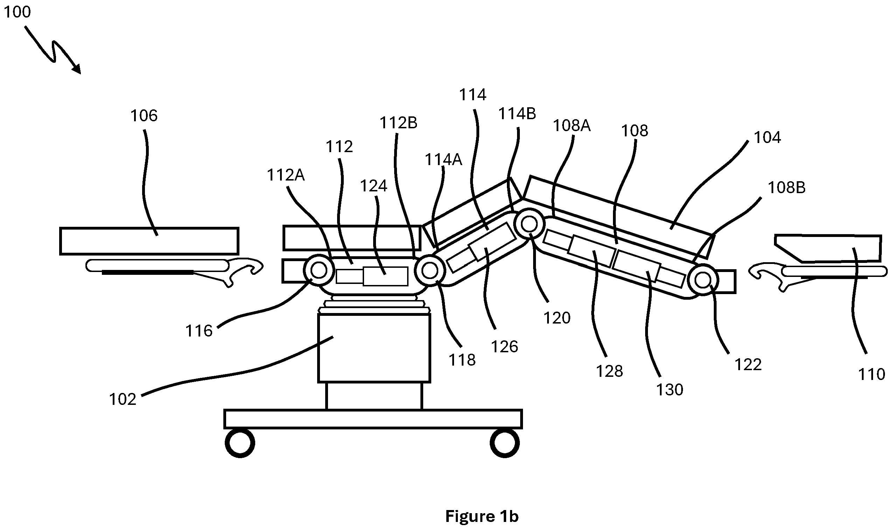

Figures 1a and 1b illustrate an example of an operating table 100 for elevating a patient's kidney region relative to their pelvic region. The operating table 100 comprises a column 102 and an operating table surface 104. The column 102 is configured to support the operating table surface 104. The operating table surface 104 is a top surface of an operating table 100 on which a patient is supported during a surgical procedure. The operating table surface 104 comprises a foot section 106, a seat section, a back section 108, and a head section 110.

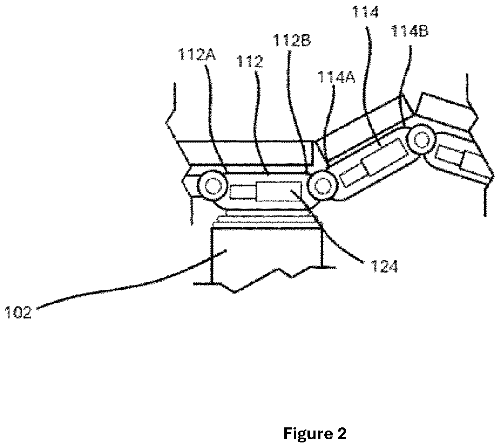

The seat section comprises a first seat portion 112 and a second seat portion 114. The column 102 is connected to the first seat portion 112 of the seat section. As shown from Figure 2, the connection between the column 102 and the first seat portion 112 is configured such that the longitudinal axis of the first seat portion 112 is perpendicular to the longitudinal axis of the column 102.

The first seat portion 112 comprises a first longitudinal end 112A and second longitudinal end 112B, the second seat portion 114 comprises a first longitudinal end 114A and second longitudinal end 114B, and the back section 108 comprises a first longitudinal end 108A and second longitudinal end 108B.

The foot section 106 is pivotably coupled to the first longitudinal end 112A of the first seat portion 112 by a joint 116. The foot section 106 is removably detachable from the first seat portion 112. The second longitudinal end 112B of the first seat portion 112 is pivotably coupled to the first longitudinal end 114A of the second seat portion 114 by joint 118. The second longitudinal end 114B of the second seat portion 114 is pivotally coupled to the first longitudinal end 108A of the back section by joint 120. The head section 110 is pivotably coupled to the second longitudinal end 108B of the back section 108 by joint 122. The head section 110 is removably detachable from the back section 108.

The operating table 100 further comprises a foot section actuator 124 housed in the first seat portion 112 and a second seat portion actuator 126 housed in the second seat portion 114. The operating table 100 further comprises a back section actuator 128 and a head section actuator 130, both of which are housed in the back section 108.

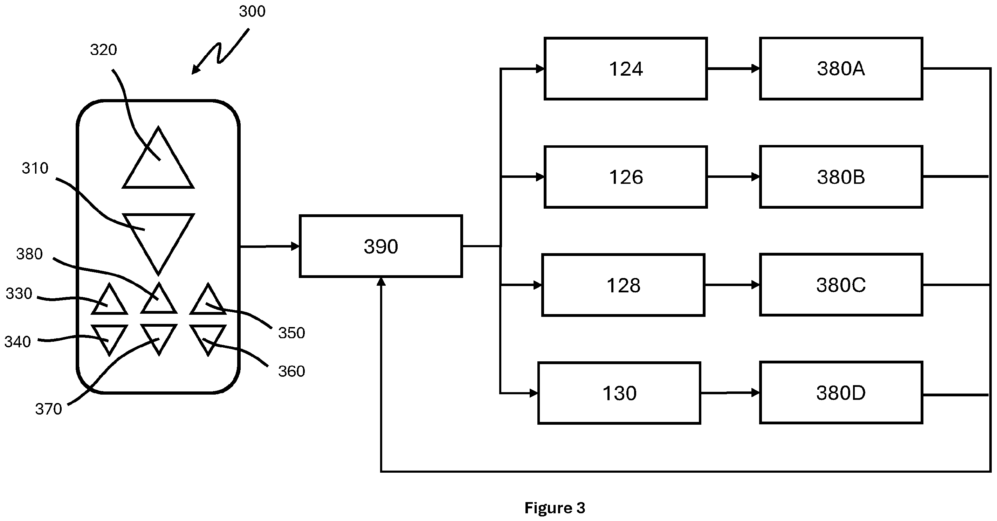

As shown in Figure 3, the operating table 100 further comprises a user interface 300, which comprises eight buttons, a first button 310, a second button 320, a third button 330, a fourth button 340, a fifth button 350, a sixth button 360, a seventh button 370, and an eighth button 380. The operating table 100 further comprises a user interface 390. The first, second, third, fourth, fifth, sixth, seventh, and eighth buttons (310, 320, 330, 340, 350, 360, 370, 380) are configured to send a first, second, third, fourth, fifth, sixth, seventh, and eighth input respectively to the controller 390. At any one time, the controller is configured to receive one input and control at least one actuator based on the received input.

The foot section actuator 124 comprises an encoder sensor 380A configured to provide positional feedback of the foot section actuator 124 to the controller 390. The second seat portion actuator 126 comprises an encoder sensor 380B configured to provide positional feedback of the second seat portion actuator 126 to the controller 390. The back section actuator 128 comprises an encoder sensor 380C configured to provide positional feedback of the back section actuator 128 to the controller 390. The head section actuator 130 comprises an encoder sensor 380D configured to provide positional feedback of the head section actuator 130 to the controller 390. At any one time, the controller 390 is configured to receive at least one input of positional feedback.

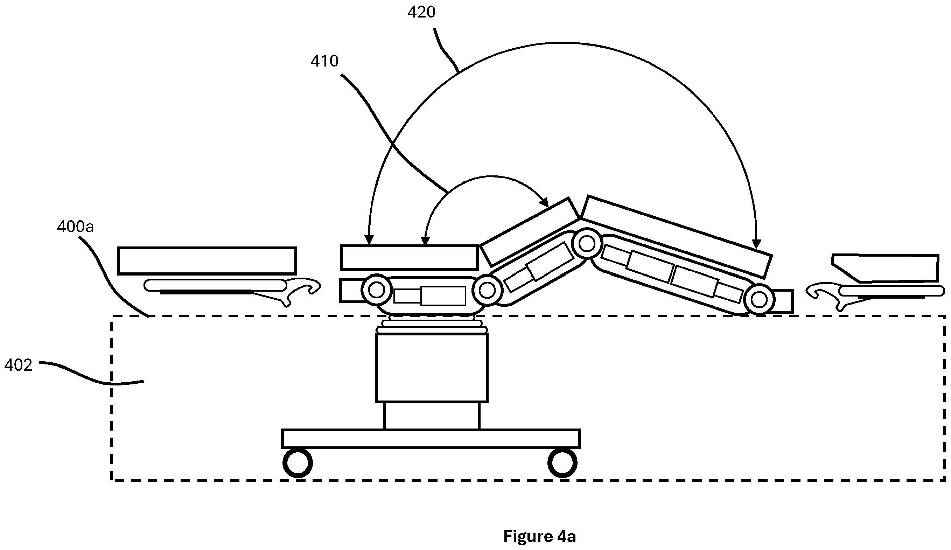

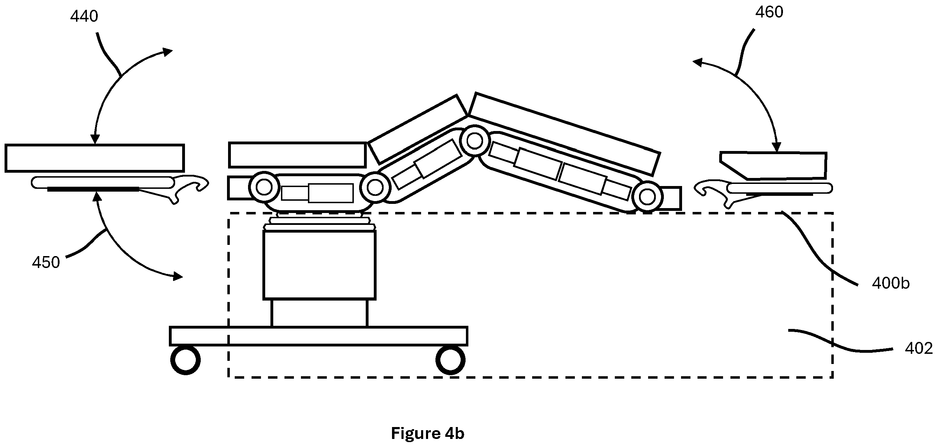

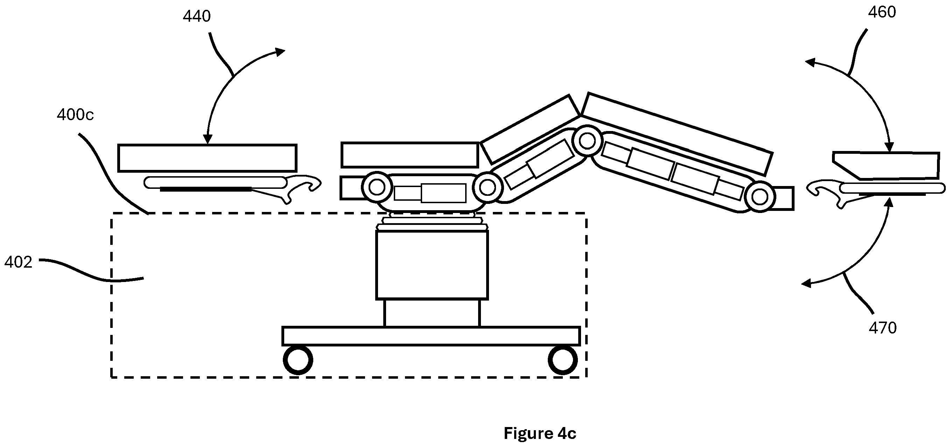

As illustrated in Figures 4a, 4b, and 4c, the operating table 100 further comprises a virtual anti-collision plane 400A, a head section virtual anti-collision plane 400B, and a foot section virtual anti-collision plane 400C. The virtual anti-collision plane 400A is defined as a plane orthogonal to the longitudinal axis of the column 102 and intersecting the connection between the column 102 and the operating table surface 104. The head section virtual anti-collision plane 400B is defined as a plane that is orthogonal to the longitudinal axis of the column 102, extending longitudinally from the first longitudinal end 112A of the first seat portion 112 towards the longitudinal end of the head section 110, and intersecting the connection between the column 102 and the operating table surface 104. The foot section virtual anti-collision plane 400C is defined as a plane that is orthogonal to the longitudinal axis of the column 102, extending longitudinally from the first longitudinal end 108A of the back section 108 towards the longitudinal end of the foot section 106, and intersecting the connection between the column 102 and the operating table surface 104.

The foot section actuator 124 is configured to pivot the foot section 106 about the first longitudinal end 112A of the first seat portion 112. The second seat portion actuator 126 is configured to pivot the second seat portion 114 about the second longitudinal end 112B of the first seat portion 112. The back section actuator 128 is configured to pivot the back section 110 about the second longitudinal end 114B of the second seat portion 114. The head section actuator 130 is configured to pivot the head section 110 about the second longitudinal end 108B of the back section 108. In this particular embodiment, the first seat portion 112 is configured to remain in a fixed position relative to the column 102.

The first 310 and second button 320 of the user interface enables a user to move the operating table 100 between a first pre-defined operating table surface configuration and a second pre-defined operating table surface configuration. The first pre-defined operating table surface configuration is defined as an operating table surface configuration in which the operating table surface is flat, as illustrated in Figure 1a. The second pre-defined operating table surface configuration is defined as an operating table surface configuration in which the second seat portion 114 is at an angle of between about 130 to about 150 degrees relative to the first seat portion 112, as illustrated in Figure 1b. In one specific example, as illustrated in Figure 4a, the second seat portion angle 410 is about 150 degrees. The second pre-defined operating table surface configuration is further defined as an operating table surface configuration in which the back section 108 is at an angle of between about 210 to about 230 degrees relative to the first seat portion 112, as illustrated in Figure 1b. In one specific example, as illustrated in figure 4a, the back section angle 420 is about 205 degrees. The second pre-defined operating table surface configuration enables a patient being supported on the operating table surface 104 to have their kidney or thorax region elevated relative to their pelvic region.

The user can configure the controller 390 to be configured such that no portion of the operating table 100 crosses the virtual anti-collision plane 400A, the head section virtual anti-collision plane 400B, or the foot section anti-collision plane 400C. The configuration of the controller by the user will depend on the type of surgery the patient receives and the required positioning of robotic surgery equipment, or the like, attached to the operating table underneath the operating table surface 104. As such, the volume 402 can remain clear of any movable portion of the operating table (other than the equipment attached thereto).

When the user presses the first button 310, the controller 390 receives the first input from the user interface 300. When the first input is received by the controller 390, the controller causes the second seat portion actuator 126 and the back section actuator 128 to move the operating table 100 towards the first operating table surface configuration. When the user presses the second button 320, the controller 390 receives the second input from the user interface 300. When the second input is received by the controller 390, the controller 390 causes the second seat portion actuator 126 and the back section actuator 128 to move the operating table 100 towards the second pre-defined operating table surface configuration.

When the user presses the third button 330, the controller 390 receives the third input from the user interface 300. When the third input is received by the controller 390, the controller 390 causes the foot section actuator 124 to move the foot section 106 increasingly towards a maximum foot section angle of 180 degrees relative to the first seat portion 112, as illustrated in Figure 4a. When the user presses the fourth button 340, the controller 390 receives the fourth input from the user interface 300. When the fourth input is received by the controller 390, the controller 390 causes the foot section actuator 124 to move the foot section 106 increasingly towards a minimum foot section angle 440 of 90 degrees relative to the first seat portion 112, as illustrated in Figure 4b.

When the user presses the fifth button 350, the controller receives the fifth input from the user interface 300. When the fifth input is received by the controller 390, the controller 390 causes the head section actuator 130 to increasingly move the head section 110 towards a maximum head section angle 460 of 90 degrees relative to the first seat portion 112, as illustrated in Figure 4b. When the user presses the sixth button 260, the controller 390 receives the sixth input from the user interface 300. When the sixth input is received by the controller 390, the controller 390 causes the head section actuator 130 to move the head section increasingly towards the minimum head section angle of 180 degrees relative to the first seat portion 112, as illustrated in Figure 4a.

When the user presses the seventh button 370, the controller 390 receives the seventh input from the user interface 300. When the seventh input is received by the controller 390, the controller 390 causes the back section actuator 128 to increasingly move the back section 108 towards a maximum back section angle 420 of 205 degrees relative to the first seat portion 112, as illustrated in Figure 4a. When the user presses the eighth button 380, the controller 390 receives the eighth input from the user interface 300. When the eighth input is received by the controller 390, the controller 390 causes the back section actuator 128 to increasingly move the back section 108 towards a minimum back section angle of 180 degrees relative to the first seat portion 112, as illustrated in Figure 1a.

When the controller 390 is configured such that the head section virtual anti-collision plane 400B is activated, and when the user presses the third button 330, the controller 390 receives the third input from the user interface 300. When this third input is received by the controller 390, the controller 390 causes the foot section actuator 124 to move the foot section 106 increasingly towards a maximum foot section angle 450 of 275 degrees relative to the first seat portion 112, as illustrated in Figure 4b.

When the controller 390 is configured such that the foot section virtual anti-collision plane 400C is activated, and when the user presses the sixth button 330, the controller 390 receives the sixth input from the user interface 300. When the sixth input is received by the controller 390, the controller 390 causes the head section actuator 130 to move the head section 110 increasingly towards a maximum head section angle 470 of 275 degrees relative to the first seat portion 112, as illustrated in Figure 4c.

The controller 390 is configured to receive at all times the positional feedback of each actuator from their respective encoder sensor. The controller 390 is further configured to ensure that no portion of the operating table 100 crosses the virtual anti-collision plane 400 based on the encoder's positional feedback.

The controller may also be configured to receive an input to determine which virtual anti-collision plane to activate. The selected virtual anti-collision plane will be dependent on the use case of the operating table.

It will be understood that the present disclosure has been described above purely by way of example, and modifications of detail can be made within the scope of the disclosure.

Each feature disclosed in the description, and where appropriate the claims and drawings may be provided independently or in any appropriate combination.

## Claims

1. An operating table for adjusting the position of a patient, comprising:
an operating table surface for supporting a patient; and
a column, the operating table surface being connected to the column;
the operating table surface comprising:
a seat section, comprising:
a first seat portion having a first longitudinal end and a second longitudinal end; and
a second seat portion having a first longitudinal end and a second longitudinal end;
wherein, the second longitudinal end of the first seat portion is pivotably coupled to the first longitudinal end of the second seat portion.

2. The operating table according to claim 1, further comprising a back section having a first longitudinal end and a second longitudinal end, wherein the first longitudinal end of the back section is pivotably coupled to the second longitudinal end of the second seat portion, preferably further comprising:
a second seat portion actuator configured to pivot the second seat portion about the second longitudinal end of the first seat portion, and
a back section actuator configured to pivot the back section about the second longitudinal end of the second seat portion.

3. The operating table according to claim 2, wherein the operating table surface further comprises a head section coupled to the second longitudinal end of the back section.

4. The operating table according to claim 3, wherein the head section is pivotably coupled to the second longitudinal end of the back section, preferably further comprising a head section actuator configured to pivot the head section about the second longitudinal end of the back section.

5. The operating table according to any preceding claim, wherein the operating table surface further comprises a foot section coupled to the first longitudinal end of the first seat portion.

6. The operating table according to claim 5, wherein the foot section is pivotably coupled to the first longitudinal end of the first seat portion, preferably further comprising a foot section actuator configured to pivot the foot section about the first longitudinal end of the first seat portion.

7. The operating table according to any preceding claim, wherein the connection between the column and the first seat portion is configured such that the longitudinal axis of the first seat portion is perpendicular to the longitudinal axis of the column.

8. The operating table according to any of claims 2 to 7, further comprising a controller, wherein the controller is configured to control one or more of the actuators to position the operating table surface.

9. The operating table according to claim 8, further comprising a virtual anti-collision plane, orthogonal to the longitudinal axis of the column and intersecting the connection between the column and the operating table surface, wherein, the controller is configured such that no portion of the operating table surface crosses the virtual anti-collision plane.

10. The operating table according to any of claims 8 or 9, wherein the controller comprises instructions for at least two pre-defined operating table surface configurations, the controller being configured to cause the actuators to configure the operating table surface to be in one of the at least two pre-defined operating table surface configurations;
wherein, the at least two pre-defined operating table surface configurations comprise:
a first pre-defined operating table surface configuration in which the operating table surface is substantially flat; and,
a second pre-defined operating table surface configuration in which the first seat portion and the second seat portion are configured such that a patient being supported on the operating table has their kidney region elevated relative to their pelvic region.

11. The operating table system according to claim 10, wherein in the second pre-defined operating table surface configuration, the second seat portion is at an angle of between about 120 degrees and about 170 degrees relative to the first seat portion.

12. The operating table system according to claim 10 or 11, the controller being configured to receive a first input and a second input, wherein:
the first input causes the controller to move the operating table towards the first pre-defined operating table surface configuration, and
the second input causes the controller to move the operating table towards the second pre-defined operating table surface configuration.

13. The operating table according to any preceding claim, wherein the foot section is removably detachable from the first seat portion.

14. The operating table according to any preceding claim, wherein the head section is removably detachable from the back section.

15. A method of configuring an operating table according to any preceding claim, comprising:
configuring the operating table surface in a first pre-defined operating table surface configuration in which the operating table surface is substantially flat; and,
configuring the operating table surface in a second pre-defined operating table surface configuration in which the first seat portion and the second seat portion are configured such that a patient being supported on the operating table has their kidney region elevated relative to their pelvic region.
